# EUROPEAN PATENT APPLICATION

(11) **EP 2 311 345 A2**
(43) Date of publication of application: **20.04.2011**
(21) Application number: 09757636.7
(22) Date of filing: 04.05.2009
(51) Int. Cl.: A46B 15/00

(54) **HAIR BRUSH**

(30) Priority: 03.06.2008 ES 200801179 U
(71) Applicant: Natural Logistics, S.L., 28224 Pozuelo de Alarcón, Madrid (ES)
(72) Inventor: SANCHEZ MARTINEZ, Alvaro, 28224 Porzuello de Alarcon (Madrid) (ES)
(74) Representative: Isern-Jara, Nuria
(86) International application number: PCT/ES2009/000234
(87) International publication number: WO 2009/147257

(57) **Abstract**

The invention relates to a hair brush comprising a handle body and a plurality of bristles disposed on an independent removable element provided with quick-coupling means for coupling/removing same in relation to the handle body. The hair brush is provided with an electrically operated and powered vibrator such that, when in operation, the vibrator performs a massaging movement at the bristle application point.

## Description

### OBJECT OF THE INVENTION

The present application is related to a hair brush that incorporates significant innovations and advantages when compared to known brushes.

More specifically, the invention relates to a hair brush comprising a handle body and a plurality of bristles, which is easy to use and offering greater hygiene.

### BACKGROUND OF THE INVENTION

It is well known that there are a wide range of hair brushes on the market and that are differentiated primarily from the aesthetic viewpoint or in the type of bristles utilized, either with respect to the hardness or the length of each one of the bristles.

However, none of the brushes known by the applicant considers the existence of an invention that has the characteristics described herein, such that it may be used by multiple users whilst ensuring hygiene.

### DESCRIPTION OF THE INVENTION

The present invention has been developed with the aim of providing a hair brush that solves the problems previously mentioned, also providing other additional advantages which will be apparent from the description detailed hereinafter.

It is therefore an object of the present invention to provide a hair brush comprising a handle body and a plurality of bristles, and it is **characterized in that** the plurality of bristles are disposed on an independent element provided with quick-coupling means for coupling same in the handle body.

Thanks to these characteristics, we obtain an original hair brush such that the user may exchange various types of different bristles depending on their use or need without having to change brush, the exchange of bristles being carried out in a simple, easy and particularly quick manner. This aspect also provides the option for multiple users to be able to utilize the same brush by simply changing the element provided with the bristles.

In a preferred embodiment, the quick-coupling elements comprise tabs that fit on the inside of a housing defined in the handle body that facilitate assembly and disassembly of the independent element quickly and easily for the user without requiring any additional element.

Preferably, the handle body is provided with a recess into which the independent element fits.

Advantageously, the edge of the front part of the handle body which defines the recess includes a sunken portion. In this way, the user, by manually applying pressure or with the aid of any pointed element can lift and remove the aforementioned independent element.

In accordance with another aspect of the invention, the independent element is formed by a body with a substantially rectangular plane from which tabs laterally protrude, which constitute the quick-coupling elements.

Another object of the invention is to provide the housing that is provided with at least one orifice through which a laser beam coming from a laser device passes through, said laser device being housed on the inside of the handle body. Thus, the comb has the capacity to provide energy effects that are well known in processes of laser phototherapy, thereby encouraging the growth of thicker, healthier and more voluminous hair. The type of laser utilized is that known as "low intensity laser",which stimulates the hair follicles to produce new hair without causing side effects.

In a preferred embodiment of the brush, an elevated section protrudes from the housing provided with a plurality of orifices through which corresponding laser beams pass, while more specifically, the orifices are located in corresponding lugs that protrude outwardly from the abovementioned elevated section, such that it prevents the beam of light from fading or losing power as occurs with the rest of the known brushes, such that each point is focused on more intensely. Similarly, another advantageous feature is the fact that it allows penetration of the laser beam through to the root of the hair itself, the point where it really has a therapeutic and beneficial effect.

In accordance with another characteristic of the invention, the brush is additionally provided with an electrically- powered vibrator device such that, when in operation, the vibrator device performs a massaging movement at the bristle application point. Additionally, the intensity of the massaging movement can be adjusted, adopting different massaging sensations.

Other characteristics and advantages of the hair brush, object of the present invention will become apparent from the description of a preferred, although not exclusive embodiment that is illustrated by way of non-limiting example in the drawings that are appended, in which:

### BRIEF DESCRIPTION OF THE DRAWINGS

Figure 1.- Perspective view of the hair brush in accordance with the present invention in a condition in which the independent removable element is separated from the handle body; and
Figure 2.- Perspective view of a second embodiment of the hair brush of the invention; and
Figure 3.- Cross-sectional view of the brush of the invention which rests on a base.

### DESCRIPTION OF A PREFERRED EMBODIMENT

As shown in the embodiments of the invention, the hair brush essentially comprises an elongated handle body 1 made, for example, from any type of plastic material and a plurality of bristles or teeth 2 arranged in an orderly manner. No further detail will be provided on the characteristics of the bristles or teeth since they are not specifically the object of the invention and they may be made by any known technique of the art. The plurality of bristles 2 are fitted into an independent removable element 3 also made, for example, from plastic material provided with quick-coupling means which are detailed hereinafter, for coupling same to the handle body 1, such that one brush can be shared amongst several users, for example, those in the same family, with each user having 10 their own independent element.

As can be seen in both embodiments, the quick-coupling elements comprise a number of tabs 4 that fit into a number of openings 5 located on the inside of the housing 6 defined in the handle body 1 where the independent element fits into place 3.Two of the tabs located on the opposite sides have a harpoon-shaped end piece ensuring that the independent removable element 3 is securely coupled during use thereof.

To facilitate removal of the independent element 3 in relation to the handle body 1 there is a sunken portion 7 with an arched shape disposed on the front edge of the recess.

The housing 6 is provided with an elevated section 8 provided with a plurality of orifices 9 through which the corresponding laser beams from the laser device pass (not shown), said laser device housed on the inside of the handle body 1 and which is powered electrically through a switch located on the outer face and powered by a rechargeable battery. Additionally, the handle body 1 has a display screen enabling the user to see the battery charge status in order to operate the laser device.

The independent removable element 3 has a longitudinal opening through which the aforementioned elevated section protrudes 8 so that the laser beams can act on the hair.

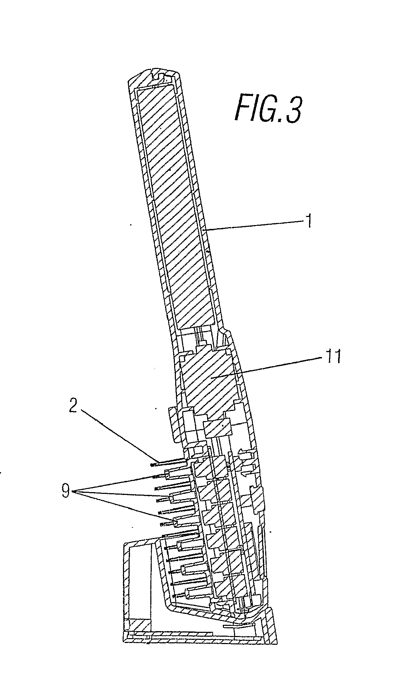

Furthermore, as can be seen from figure 3, the brush is provided with a vibrator device 11 housed on the inside of the handle body 1 which is electrically powered by the battery such that, when in operation said vibrator device 11 performs a massaging movement at the bristle application point which encourages the stimulation of blood flow to the scalp. This device can be operated via a push-button also located on the outer part of the brush body.

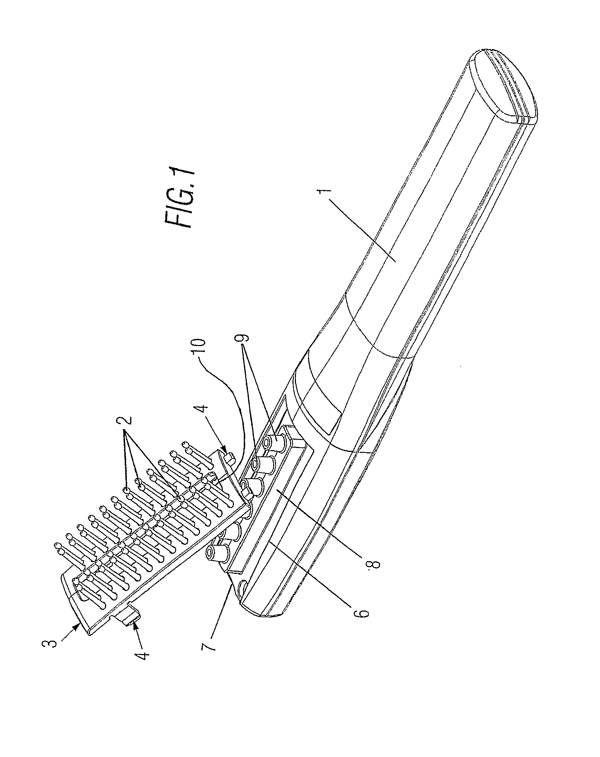

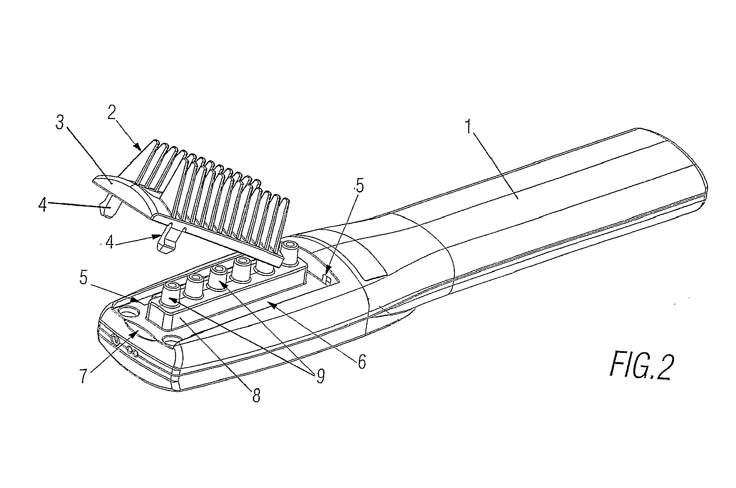

The existing difference between the two embodiments shown in figures 1 and 2 lies in the type of bristle provided in the independent element, maintaining the same numerical references in both embodiments for those common elements.

The details, shapes, dimensions and other accessory elements , as well as the materials used to manufacture the hair brush of the invention may be suitably replaced by others which are technically equivalent, provided they do not depart from the essential nature of the invention or from the scope defined by the claims appended hereinafter.

## Claims

1. A hair brush comprising a handle body and a plurality of bristles, **characterized in that** the plurality of bristles are disposed on an independent removable element provided with quick-coupling means for coupling same and removing in relation to the handle body.

2. A hair brush according to claim 1, **characterized in that** the quick-coupling elements comprise tabs that fit into the corresponding openings located on the inside of the housing defined on the handle body.

3. A hair brush according to any of the preceding claims, **characterized in that** the handle body is provided with a recess into which the independent element fits.

4. A hair brush according to any of the claims, **characterized in that** the edge of the front part of the handle body that defines the recess includes a sunken portion.

5. A hair brush according to claims 1 and 2, **characterized in that** the independent element is formed by a body with a substantially rectangular plane with laterally projecting tabs which constitute the quick-coupling elements.

6. A hair brush according to claim 2, **characterized in that** the housing is provided with at least one orifice through which a laser beam coming from an electrically-operated laser device passes, said laser device being housed on the inside of the handle body.

7. A hair brush according to claim 6, **characterized in that** an elevated section protrudes from the housing provided with a plurality of orifices through which the corresponding laser beams pass.

8. A hair brush according to claims 5 and 7, **characterized in that** the independent removable element has an opening through which the aforementioned elevated section protrudes.

9. A hair brush according to claim 7, **characterized in that** the orifices are located on the corresponding lugs that protrude outwardly from the aforementioned elevated section.

10. A hair brush according to claim 1, **characterized in that** it is provided with an electrically operated and powered vibrator device such that, when in operation, said device performs a massaging movement at the bristle application point.

11. A hair brush according to claim 10, **characterized in that** the vibrator device is adjustable in the intensity of the massaging movement.
